# EUROPEAN PATENT APPLICATION

(11) **EP 4 321 557 A1**
(43) Date of publication of application: **14.02.2024**
(21) Application number: 22784637.5
(22) Date of filing: 31.03.2022
(51) Int. Cl.: C08J 3/12, C08L 67/04, C08L 101/16, C08G 63/06, C12P 7/62

(54) **MICROPARTICLES CONTAINING POLYHYDROXYALKANOIC ACID (PHA) AND METHOD FOR PRODUCING SAME**

(30) Priority: 06.04.2021 JP 2021064686
(71) Applicant: Fuence Co., Ltd., Tokyo 150-0012 (JP)
(72) Inventor: INOUE, Kozo, Tokyo 150-0012 (JP); KUMAR, K. Sudesh, Penang 11700 (MY); MIYAUCHI, Hironaga, Tokyo 150-0012 (JP)
(74) Representative: Script IP Limited
(86) International application number: PCT/JP2022/016649
(87) International publication number: WO 2022/215653

(57) **Abstract**

The present invention provides a microparticle comprising 3-hydroxybutanoic acid (3-HB) as a repeating unit of polyhydroxyalkanoic acid (PHA), a biodegradable polymer, and having a particle size of 0.2 um to less than 10 um, and a method for producing the microparticle. The microparticle of the present invention can be used in a wide range of applications, including pharmaceutical applications, because of its biodegradability, excellent processability, and biocompatibility.

## Description

### Technical Field

The present invention relates to a microparticle comprising polyhydroxyalkanoic acid (PHA), the microparticle comprising a unit of 3-hydroxybutanoic acid (3-HB) as a repeating unit of polyhydroxyalkanoic acid (PHA), and having a particle size of 0.2 um to less than 10 um, and a method for producing the microparticle.

### Background Art

Microparticles consisting of synthetic resins have become indispensable materials in many fields since they have various applications in a medical field of, for example, a drug delivery system (DDS) and a medical diagnostics test particle, in addition to their uses as modifiers such as plastic resin modifiers and cosmetic modifiers, additives such as paint additives, toner additives, and cosmetic additives, and fillers such as spacers for liquid crystal, chromatographic fillers, and adhesive tape fillers. In addition to problems of fluctuating raw material oil prices and inconsistent raw material supply, however, microparticles made of synthetic resins have a negative impact on the environment, producing greenhouse gases and causing various problems, such as the flow of waste water into rivers and oceans directly or through wastewater treatment plants after being used in the production process or after production. Therefore, there is an urgent need to solve these problems on a global scale.

In particular, contamination of microplastics in the ocean has recently become a problem. For example, when plastic waste is crushed by waves and UV rays, it becomes microplastic of 5 millimeters long or less, and such microplastics accumulate in fish bodies. In 50 years, plastic waste in the sea would exceed the total weight of fish, and reducing plastic waste has become an urgent problem for human beings. Especially, the use of microparticles consisting of synthetic resins has already been greatly restricted because they are themselves microplastics. Therefore, in industrial fields where the use of microparticles consisting of synthetic resins is essential, there is an urgent need to replace conventional synthetic resins as the raw material for microparticles (Non-patent Document 1).

Although the microparticles consisting of synthetic resins have great expectations for the purpose of application to, for example, drug delivery systems (DDS) in the medical field as described above, in addition to the problems of the aforementioned microparticles consisting of synthetic resins in the medical field, it has also become necessary to satisfy conditions such as biocompatibility to enable safe use in vivo (Non-patent Document 2).

As one of the solutions to these problems, the use of biodegradable biopolymers as raw materials for microparticles has been proposed. Although the development of microparticles using biopolymers such as polylactic acid (PLA), polyhydroxyalkanoic acid (PHA), and cellulose has already been proposed (Patent Documents 1 and 2), it has been pointed out that the microparticles is "biodegradable" not in a high-temperature and high-humidity environment such as compost but in an actual river or marine environment only when polyhydroxyalkanoic acid (PHA) and cellulose are used as raw materials.

Meanwhile, the production of microparticles using polyhydroxyalkanoic acid (PHA) as a biodegradable biopolymer as a raw material for microparticles has been mainly developed using poly-3-hydroxybutanoic acid (3-PHB). Patent Document 3 describes syringeable microparticles consisting of a biocompatible and biodegradable polymer, in which a copolymer of 3-hydroxybutanoic acid and 4-hydroxybutanoic acid, poly(4-hydroxybutyrate-co-3-hydroxybutyrate), is used as the polymer. Patent Document 4 discloses a cosmetic composition in the form of microparticles containing polyhydroxyalkanoate (PHA), and poly-3-hydroxybutyrate (PHB), poly-3-hydroxyhexanoate (PHH), and poly(3-hydroxybutyrate-co-4-hydroxybutyrate) are given as examples of the PHA. In addition, Patent Document 5 discloses porous resin particles containing polyhydroxyalkanoate, and suitable examples of the polyhydroxyalkanoate include poly(3-hydroxybutyrate-co-3-hydroxyhexanoate), a copolymer of a 3-hydroxybutyrate unit and a 3-hydroxyhexanoate unit. Patent Document 6 describes a nonwoven fabric containing polyhydroxyalkanoate, preferably poly-4-hydroxybutyrate and a copolymer thereof, through a dry spinning process, with microfibers having a specific average diameter and a specific burst strength.

However, because poly-3-hydroxybutanoic acid has problems with physical properties such as brittleness and hardness, as well as the cost of production and purification, the practical use of microparticles with desired physical properties using poly-3-hydroxybutanoic acid as the main raw material has not yet been achieved.

### Prior Art Documents

### Patent Documents

Patent Document 1: Japanese Patent No. 5133478
Patent Document 2: US Patent Application Publication No. 2006/0177513
Patent Document 3: US Patent No. 10463619
Patent Document 4: International Publication No. WO 2018/178899
Patent Document 5: International Publication No. WO 2017/056908
Patent Document 6: Japanese unexamined Patent Application Publication (Translation of PCT Application) No. 2013-534978

### Non-patent Documents

Non-patent Document 1: JFE Techno-Research Corporation. "FY 2016 Report on a Survey Commissioned by the Ministry of Economy, Trade and Industry; FY 2016 Report on Safety Measures for Chemical Substances (Fact-finding Survey on Domestic Microplastic Emission)," February 2017
Non-patent Document 2: Microparticles, Microspheres, and Microcapsules for Advanced Drug Delivery. Sci. Pharm. 2019, 87, 20

### Summary of the Invention

### Object to be Solved by the Invention

In order to provide microparticles that can be used for a wide range of applications, including medical applications, there has been a demand for a biodegradable polymer suitable for producing a microparticle having physical properties required in each application, in particular, an appropriate particle size distribution and retention of other substances as important physical properties, as well as a demand for establishing a method for more easily producing a microparticle having physical properties that can be used in a wide range of applications from the biodegradable polymer.

### Means to Solve the Object

As a result of studies to solve the above problems, the present inventors have found that a microparticle containing 3-hydroxybutanoic acid (3-HB) as a repeating unit of polyhydroxyalkanoic acid (PHA) and having a particle size of 0.2 um to less than 10 um has desired physical properties, based on the finding that the polyhydroxyalkanoic acid (PHA) having excellent melt flowability can be produced by a method including the steps of: providing a microorganism that produces polyhydroxyalkanoic acid (PHA); proliferating the microorganism in a medium; causing an animal to ingest a proliferated microorganism; and recovering the polyhydroxyalkanoic acid (PHA) from the excreta of the animal, thus completing the present invention (for the above finding, see Japanese Patent Application No. 2019-086889 filed on April 26, 2019).

In other words, the present inventors have confirmed that a microparticle containing polyhydroxyalkanoic acid (PHA), the microparticle comprising a unit of 3-hydroxybutanoic acid (3-HB) as a repeating unit thereof and having a particle size of 0.2 um to less than 10 um has the desired physical properties, thus completing the present invention.

The present invention is as specified by particular matters below.
(1) A microparticle comprising polyhydroxyalkanoic acid (PHA), the microparticle comprising 3-hydroxybutanoic acid (3-HB) as a repeating unit of polyhydroxyalkanoic acid (PHA), and having a particle size of 0.2 um to less than 10 um.
(2) The microparticle according to (1), wherein the microparticle has a particle size of 7 um or less.
(3) The microparticle according to (1) or (2), wherein the polyhydroxyalkanoic acid (PHA) further comprises 3-hydroxyhexanoic acid (3-HH) as a repeating unit thereof.
(4) The microparticle according to (3), wherein a proportion of the 3-hydroxyhexanoic acid (3-HH) is 27% by weight or less with respect to a total weight of the repeating unit of polyhydroxyalkanoic acid (PHA).
(5) The microparticle according to (3) or (4), wherein the polyhydroxyalkanoic acid (PHA) comprises a copolymer of the 3-hydroxybutanoic acid (3-HB) and the 3-hydroxyhexanoic acid (3-HH).
(6) The microparticle according to any one of (1) to (5), wherein the polyhydroxyalkanoic acid (PHA) further comprises a unit of 4-hydroxybutanoic acid (4-HB) as a repeating unit thereof.
(7) The microparticle according to (6), wherein a proportion of the 4-hydroxybutanoic acid (4-HB) is 40 to 50% by weight with respect to a total weight of the repeating unit of polyhydroxyalkanoic acid (PHA).
(8) The microparticle according to (6) or (7), wherein the polyhydroxyalkanoic acid (PHA) comprises a copolymer of the 3-hydroxybutanoic acid (3-HB) and the 4-hydroxybutanoic acid (4-HB).
(9) The microparticle according to any one of (1) to (8), wherein the polyhydroxyalkanoic acid (PHA) has an average molecular weight (Mw) of 100,000 to 1,300,000.
(10) The microparticle according to any one of (1) to (9), wherein the polyhydroxyalkanoic acid (PHA) has a melting point of 55°C or more to 170°C or less.
(11) The microparticle according to any one of (1) to (10), wherein the microparticle comprises a resin other than the polyhydroxyalkanoic acid (PHA).
(12) The microparticle according to (11), wherein the resin other than the polyhydroxyalkanoic acid (PHA) is a biodegradable resin.
(13) The microparticle according to any one of (1) to (12), wherein the microparticle is spherical.
(14) The microparticle according to any one of (1) to (13), wherein the microparticle is porous.
(15) The microparticle according to any one of (1) to (14), wherein the microparticle retains other substances on a surface and/or inside thereof.
(16) The microparticle according to any one of (1) to (15), wherein the microparticle has a 10% compressive strength of 0.23 to 2.20 MPa.
(17) The microparticle according to any one of (1) to (16), wherein the microparticle is dispersible in an aqueous solvent.
(18) A method for producing the microparticle according to any one of (1) to (17), comprising:
   Step 1: a step of providing a microorganism that produces polyhydroxyalkanoic acid (PHA);
   Step 2: a step of proliferating the microorganism of Step 1 in a medium;
   Step 3: a step of causing an animal to ingest a proliferated microorganism;
   Step 4: a step of recovering and purifying the polyhydroxyalkanoic acid (PHA) from excreta of the animal of Step 3; and
   Step 5: a step of making microparticles from the polyhydroxyalkanoic acid (PHA) obtained in Step 4.
(19) The method for producing the microparticle according to (18), wherein Step 5 is a step of micronizing a resin composition comprising the polyhydroxyalkanoic acid (PHA) obtained in Step 4.

### Effect of the Invention

According to the present invention, a microparticle comprising polyhydroxyalkanoic acid (PHA) comprises 3-hydroxybutanoic acid (3-HB) as a repeating unit of polyhydroxyalkanoic acid (PHA), and therefore, the microparticle comprising such polyhydroxyalkanoic acid (PHA) has excellent biodegradability in a natural environment and processability. According to the present invention, the microparticle comprising the polyhydroxyalkanoic acid (PHA) and having a particle size of 0.2 um to less than 10 um may have physical properties that can be used in a wide range of applications, such as melting point, particle size, porosity, compressive strength, and substance retention. The present invention also provides a simple method for producing the microparticle comprising such polyhydroxyalkanoic acid (PHA) .

Thus, the present invention provides a microparticle comprising the polyhydroxyalkanoic acid (PHA), the microparticle having physical properties that can be used in a wide range of applications with biodegradability, excellent processability, and biocompatibility.

Since the microparticle according to the present invention can be provided as a microparticle with excellent biodegradability in a natural environment, it can contribute to the resolution of marine pollution, microplastic problems, or the like. In addition, the microparticle is expected to reduce incineration and environmental impact since it allows the material to be biodegraded in disposal. Furthermore, since the microparticle according to the present invention may have physical properties that can be used for a wide range of applications in addition to the biocompatibility and biodegradability possessed by polyhydroxyalkanoic acid (PHA), it is highly likely to be used for a wide range of medical applications in addition to conventional applications.

### Brief Description of Drawings

[Figure 1] Figure 1 is a conceptual diagram showing a basic configuration of an electrospray deposition device.
[Figure 2] Figure 2 is a SEM observation image of microparticles produced from P (3-HB) in Example 1.
[Figure 3] Figure 3 is a SEM observation image of porous microparticles produced from P (3-HB) in Example 2.
[Figure 4] Figure 4 is a SEM observation image of microparticles produced from P (3-HB-co-3-HH) in Example 3.
[Figure 5] Figure 5 is a SEM observation image of microparticles produced from P (3-HB-co-4-HB) in Example 4.
[Figure 6] Figure 6 is a SEM observation image showing that microparticles produced from P (3-HB) in Example 5 have retained silica particles on the surface thereof.
[Figure 7] Figure 7 is a SEM observation image showing that microparticles produced from P (3-HB) in Example 5 have retained silica particles inside.
[Figure 8] Figure 8 is a BSE-SEM observation image showing that microparticles produced from P (3-HB) in Example 5 have retained silica particles.

### Mode of Carrying Out the Invention

Next, specific embodiments, including the best mode for carrying out the present invention, will be described.

### [Polyhydroxyalkanoic Acid (PHA)]

Polyhydroxyalkanoic acid (PHA) is a polyester of hydroxyalkanoic acid given as an example in chemical formula (1) below and a biodegradable polymer.

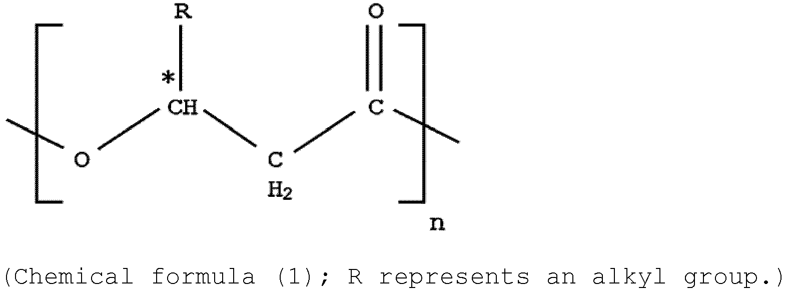

A unit of 3-hydroxyalkanoic acid (3-HA) as a repeating unit of the polyhydroxyalkanoic acid (PHA) according to the present invention is described below as chemical formula (2), and a unit of 4-hydroxyalkanoic acid (4-HA) as chemical formula (3) .

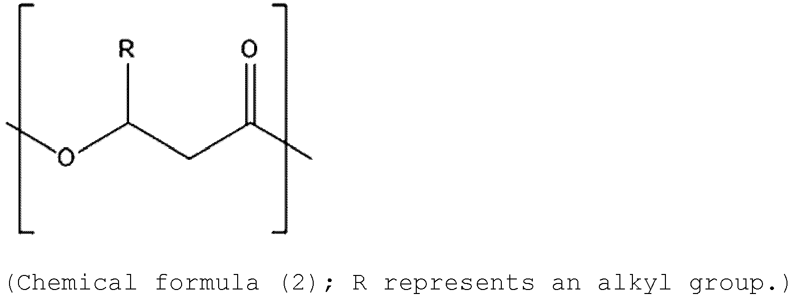

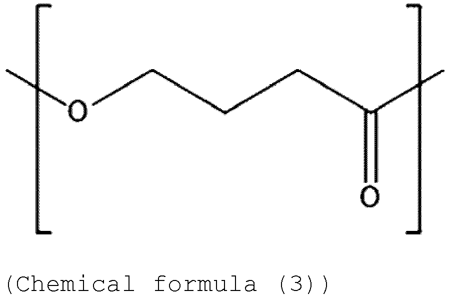

Examples of the alkyl group (R) in the unit of 3-hydroxyalkanoic acid (3-HA) include a methyl group, an ethyl group, a propyl group, a butyl group, a pentyl group, a hexyl group, a heptyl group, an octyl group, a nonyl group, a decyl group, an undecyl group, a dodecyl group, and a tridecyl group. The repeating unit of the polyhydroxyalkanoic acid (PHA) according to the present invention comprises 3-hydroxybutanoic acid (3-HB: chemical formula (4) below) in which an alkyl group described below is a methyl group. In another embodiment of the present invention, the repeating unit of the polyhydroxyalkanoic acid (PHA) contains 3-hydroxyhexanoic acid (3-HH: chemical formula (5) below) in which the alkyl group is a propyl group. In the case of comprising units of the 3-hydroxybutanoic acid (3-HB) and 3-hydroxyheptanoic acid (3-HH) as its repeating units, the polyhydroxyalkanoic acid (PHA) according to the present invention preferably comprises them as a copolymer of the 3-hydroxybutanoic acid (3-HB) and 3-hydroxyhexanoic acid (3-HH), P (3-HB-co-3-HH).

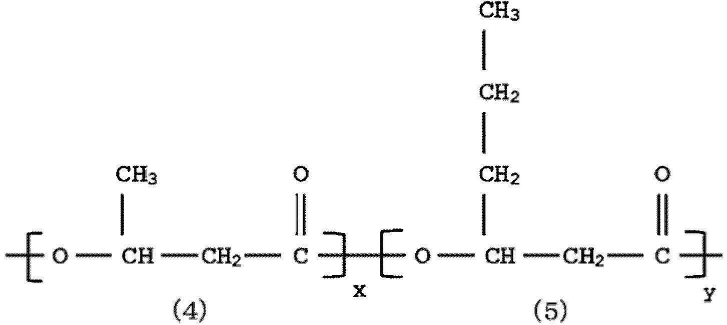

In another embodiment of the polyhydroxyalkanoic acid (PHA) according to the present invention, the proportion of 3-hydroxyhexanoic acid (3-HH) in the repeating unit of the polyhydroxyalkanoic acid (PHA) according to the present invention is 30% by weight or less, preferably 27% or less, with respect to the total amount of the repeating unit of the polyhydroxyalkanoic acid (PHA). When the proportion of the 3-hydroxyhexanoic acid (3-HH) is 27% or less with respect to the total amount of the repeating unit of the polyhydroxyalkanoic acid (PHA), it is possible to produce a porous microparticle, but when the 3-hydroxyhexanoic acid (3-HH) is comprised in an amount exceeding 27%, it may be difficult to produce a porous microparticle.

In another embodiment of the polyhydroxyalkanoic acid (PHA) according to the present invention, the repeating unit of the polyhydroxyalkanoic acid (PHA) according to the present invention comprises 4-hydroxybutanoic acid (4-HB), which is described in chemical formula (3) above. In the case of comprising a unit of 4-hydroxybutanoic acid (4-HB) as its repeating unit, the polyhydroxyalkanoic acid (PHA) according to the present invention preferably comprises it as a copolymer of the 3-hydroxybutanoic acid (3-HB) and 4-hydroxybutanoic acid (4-HB), P (3-HB-co-4-HB), given as an example below.

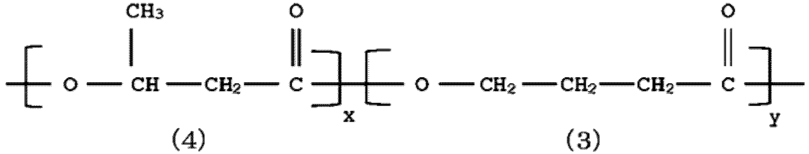

The proportion of the 4-hydroxybutanoic acid (4-HB) in the repeating unit of the polyhydroxyalkanoic acid (PHA) according to the present invention is 40 to 50% by weight, preferably 40 to 45%, and more preferably 40 to 42%, with respect to the total amount of the repeating unit of the polyhydroxyalkanoic acid (PHA). In a case where the 4-hydroxybutanoic acid (4-HB) is comprised in a proportion within the above-mentioned range with respect to the total amount of the repeating unit of the polyhydroxyalkanoic acid (PHA), it is possible to produce microparticles that can be widely used in medical applications since they are expected to have good biocompatibility and biodegradability. However, in a case where the 4-hydroxybutanoic acid (4-HB) is comprised in a proportion out of the above-mentioned range, it may be difficult to produce microparticles that can be widely used in medical applications because they are unlikely to have good biocompatibility and biodegradability.

In another embodiment of the polyhydroxyalkanoic acid (PHA) according to the present invention, the polyhydroxyalkanoic acid (PHA) has a weight average molecular weight of 1.0 × 10⁵ to 13.0 × 10⁵ g/mol, preferably 3.0 × 10⁵ to 10.0 × 10⁵ g/mol, and more preferably 3.0 × 10⁵ to 8.0 × 10⁵ g/mol. When the weight average molecular weight of the polyhydroxyalkanoic acid (PHA) is in the above-mentioned range, it is possible to provide polyhydroxyalkanoic acid (PHA) having controllable solubility in a solvent, hardness and softness in the form of a microparticle, heat resistance, and durability. However, when the weight average molecular weight is out of the above-mentioned range, such effects may not be obtained.

In another embodiment of the polyhydroxyalkanoic acid (PHA) according to the present invention, the polyhydroxyalkanoic acid (PHA) has a melting point of 55°C or more and 170°C or less, preferably 60°C to 160°C, and more preferably 80°C to 120°C. By setting the polyhydroxyalkanoic acid (PHA) melting point within the above range, it is possible to provide polyhydroxyalkanoic acid (PHA) capable of producing microparticles suitable for conditions in various applications. Note that the polyhydroxyalkanoic acid (PHA) melting point can be measured by any method, for example, by DSC analysis.

### [Method for Producing Polyhydroxyalkanoic Acid (PHA)]

The method for producing polyhydroxyalkanoic acid (PHA) according to the present invention is not particularly limited and may be any production method as long as polyhydroxyalkanoic acid (PHA) having the characteristics of the polyhydroxyalkanoic acid according to the present invention can be obtained.

For example, an embodiment of the method for producing polyhydroxyalkanoic acid (PHA) according to the present invention may include the following steps:
Step 1: a step of providing a microorganism that produces polyhydroxyalkanoic acid (PHA);
Step 2: a step of proliferating the microorganism of Step 1 in a medium;
Step 3: a step of causing an animal to ingest a proliferated microorganism; and
Step 4: a step of recovering and purifying the polyhydroxyalkanoic acid (PHA) from excreta of the animal in Step 3.

The polyhydroxyalkanoic acid (PHA) according to the present invention is preferably produced using a microorganism, and examples thereof include a microorganism capable of producing polyhydroxyalkanoic acid, such as *Bacillus megaterium, Cupriavidus necator, Ralstonia eutropha,* and *Alcaligenes latus.* Among them, *Cupriavidus necator* is particularly preferred.

The microorganism is preferably one in which a gene involved in the synthesis of polyhydroxyalkanoic acid (PHA) has been deleted or introduced. For example, it is preferable to use a microorganism in which an acetoacetyl-CoA reductase gene is deleted. It is also preferable to introduce a hydroxyalkanoate synthase gene or enoyl-CoA hydratase gene. Accordingly, the content of the unit of 3-hydroxyhexanoic acid (3-HB) comprised in the polyhydroxyalkanoic acid can be increased. In addition, it is possible to produce a copolymer P (3HB-co-3HH) consisting of 3-hydroxybutanoic acid (3-HB) and 3-hydroxyhexanoic acid (3-HH), which has high melt flowability and excellent processability.

The medium to be used for culturing microorganisms is not particularly limited as long as the microorganisms can proliferate in the medium. For example, the medium contains, as carbon sources, an alcohol such as methanol, ethanol, and butanol; a fatty acid including a saturated or unsaturated fatty acid such as acetic acid, propionic acid, hexanoic acid, octanoic acid, decanoic acid, lauric acid, oleic acid, palmitic acid, linoleic acid, linolenic acid, and myristic acid; a saccharide such as glucose and fructose; an organic acid such as lactic acid; and fat or oil containing a large amount of a saturated or unsaturated fatty acid having 10 or more carbon atoms. Examples of the fat or oil include a vegetable oil such as coconut oil, palm kernel oil, palm oil, palm olein, rapeseed oil, soybean oil, rice oil, and sesame oil; an animal fat or oil such as lard and beef tallow; and fish oil. Note that the fat or oil before purification and waste cooking oil can also be used. Preferred fat or oil to be added to the medium as a carbon source is palm kernel oil or palm oil containing lauric acid. The polyhydroxyalkanoic acid (PHA) content can be increased by inclusion of palm kernel oil or palm oil.

The microbial culture conditions for producing the polyhydroxyalkanoic acid (PHA) according to the present invention are preferably aerobic conditions. If necessary, a nitrogen source or an inorganic substance is optionally added. Examples of the nitrogen source include an ammonium salt such as ammonia, ammonium chloride, ammonium sulfate, and ammonium phosphate. Examples of the inorganic substance include monopotassium phosphate, dipotassium phosphate, magnesium phosphate, magnesium sulfate, and sodium chloride.

The culture temperature is preferably 20°C to 40°C, and more preferably 25°C to 35°C. The culture time is not particularly limited but is preferably 48 to 72 hours.

In the method for producing the polyhydroxyalkanoic acid (PHA) according to the present invention, the content of 3-hydroxyhexanoic acid (3-HH) in the copolymer of the 3-hydroxybutanoic acid (3-HB) and the 3-hydroxyhexanoic acid (3-HH) can be controlled by regulating expression levels of the acetoacetyl-CoA reductase gene and the enoyl-CoA hydratase gene.

In addition, the 3-hydroxyhexanoic acid (3-HH) content in the copolymer of the 3-hydroxybutanoic acid (3-HB) and the 3-hydroxyhexanoic acid (3-HH) can also be controlled by controlling the remaining amount of carbon sources, adjusting the concentration of inorganic components in the culture solution, and adjusting the volume of oxygen aeration and the culture time.

Although methods of recovering and purifying polyhydroxyalkanoic acid (PHA) are not particularly limited, examples thereof include a method of recovering polyhydroxyalkanoic acid (PHA) from the culture medium by centrifugation and extracting it with a solvent or the like; and a method of recovering polyhydroxyalkanoic acid (PHA) as excreta by causing an animal to digest and absorb the microorganisms. From the viewpoint of easy concentration of the polyhydroxyalkanoic acid (PHA), a method of causing an animal to digest and absorb the microorganisms to recover granular polyhydroxyalkanoic acid (PHA) contained in excreta is preferred.

Example of the above animal include an animal such as rodent, goat, sheep, cattle, bird, an aquatic organism, a beetle, and an insect. Among them, larvae of beetles such as mealworms are preferred, and 35 day-old beetles eating housefly (*Tenebrio molitor* larvae) are more preferred.

The polyhydroxyalkanoic acid (PHA) can be recovered by feeding the above-mentioned microorganisms to larvae of mealworms or the like, followed by collection of fecal pellets, sieving with a mesh, washing with water, a base such as sodium hydroxide, and drying.

Specific aspects of the method for producing the polyhydroxyalkanoic acid (PHA) are given as examples below.

### (1) Method for Producing P (3-HB)

After preculturing with Cupriavidus necator/H16, the culture was transferred to a 500 mL conical flask containing 10 g/L of palm oil, 0.54 g/L of urea, and 100 µL of an MM composition solution (the composition is described in (3) below), and cultured by shaking at 30°C for 24 hours at 200 rpm. After culturing, the cells were lyophilized, and about 5 g was dissolved in 500 mL of chloroform and stirred at room temperature for five days. The cell residue was separated from the solution with a filter. The solution was concentrated by a rotary evaporator, added dropwise to cold methanol, and stirred for about two hours until a precipitate was formed, thereby purifying poly 3-hydroxybutanoic acid, P (3-HB), containing no 3-hydroxyhexanoic acid (3-HH) as polyhydroxyalkanoic acid (PHA). The resulting precipitate was filtered in vacuum through a 0.2 um PTFE filter and dried.

### (2) Method for Producing P (3-HB-co-3-HH)

### (a) Preparation of mineral medium for production of P (3HB-co-3HH)

A mineral medium for the production of P (3HB-co-3HH) consisted of 4.0 g/L of NaH₂PO₄, 4.6 g/L of Na₂HPO₄, 0.45 g/L of K₂SO₄, 0.39 g/L of MgSO₄, 62 mg/L of CaCl₂, and 1 mL/L of trace element solution (the trace element solution contained 15 g/L of FeSO₄·7H₂O, 2.4 g/L of MnSO₄·H₂O, 2.4 g/L of ZnSO₄·7H₂O, and 0.48 g/L of CuSO₄·5H₂O, all of which had been dissolved in 0.1 M HCl). The pH of the medium was adjusted to 7.0 before sterilization by autoclaving.

### (b) Biosynthesis of P (3HB-co-3HH) using 13-L fermenter

Biosynthesis of P (3HB-co-3HH) was carried out using Cupriavidus necator into which a polyhydroxyalkanoate synthase gene had been introduced.

First, Cupriavidus necator transfected with a gene encoding polyhydroxyalkanoate synthase was streaked onto an agar plate and cultured at 30°C for 24 hours. Next, as a pre-culture, a 50 mL culture solution was inoculated with the Cupriavidus necator twice using a inoculation loop, and the culture solution was shaken in an incubator shaker at 30°C for eight hours until the OD600 nm of the culture solution reached 4. About 3 mL of the culture solution was inoculated into 100 mL of a mineral medium supplemented with 0.54 g/L of urea, 0.39 g/L of MgSO₄, 62 mg/L of CaCl₂, 1 mL/L of trace element solution, and 1% by mass of crude palm kernel oil. The crude palm kernel oil was autoclaved before addition to the mineral medium. The mineral medium was further cultured for 18 hours and inoculated into a 6 L fermenter. The morphology of the inoculated Cupriavidus necator was checked before transfer to the fermenter (10% v/v). The temperature of the culture medium was maintained at 30°C while the pH of the medium was set at 7.0 ± 0.1 by the addition of 3 M NaOH and 3 M H₃PO₄. Stirring was performed using a Rushton turbine at a stirring rate of 200 to 900 rpm. Air was supplied at 1 vvm (air volume/fermenter working volume/ min) through a filter cartridge (Sartorius stedim, Germany) to maintain the dissolved oxygen concentration at 40% or more. MgSO₄·7H₂O was added at the 18th hour, and urea every 6 hours after culturing. Trace elements were added at 1 mL during planting and at 18 hours of culturing. Crude palm kernel oil was supplied at a concentration of 10 g/L to 20 g/L every six hours, depending on the oil consumption by microorganisms. Sampling was performed every six hours to determine the residual oil content, wet cell weight, and optical density of the bacterial cultures. Culture times ranged from 48 hours to 72 hours depending on bacterial proliferation.

### (c) Biological recovery of P (3HB-co-3HH)

Thirty five day-old mealworms (*Tenebrio molitor* larvae) were reared in plastic containers at ambient temperatures (about 25°C). Dry microorganisms containing the above P (3HB-co-3HHx) were fed to 100 g of the reared mealworms.

The amount of microorganisms fed was based on the weight of the mealworms (5% per day of body weight). Before feeding a new batch of microorganisms, mealworms' fecal pellets were collected and sieved using meshes of sizes 0.50 mm and 0.25 mm. Through double sieving, it was possible to remove other impurities and to facilitate the subsequent washing step.

### (d) Purification of P (3HB-co-3HH) using distilled water

About 10% (w/v) of the fecal pellets were each added to tap water to a concentration of 100 g/L. The fecal pellet suspension was rinsed several times and allowed to settle before discarding the supernatant. The supernatant was removed, and the recovered P (3HB-co-3HH) was dried in an oven at 50°C to a constant mass.

The dried P (3HB-co-3HH) was further rinsed in 0.25 M NaOH for one hour, the mixture was allowed to settle to remove the supernatant, and the collected pellets were stirred in tap water for another one hour until the pH dropped below 9.5. Then, the recovered P (3HB-co-3HH) granules were dried to a constant mass in an oven at 50°C to recover the desired P (3HB-co-3HH).

### (3) Method for Producing P (3HB-co-4HB)

With Cupriavidus necator Re2058/pHT1phaCCs, an NR agar medium (with 50 ug/mL kanamycin) was plated at 30°C and grown. The grown cells were collected and transferred to a 50 ml MM medium and cultured at 48°C at 200 rpm for 48 hours. After culturing, the cells were collected by centrifugation at 8,000 rpm for 10 minutes and lyophilized for two days. P (3HB-co-4HB) was extracted with chloroform in the same manner as in (1) above.

### Composition of MM medium

Fructose: 10 g/L
Sodium 4-hydroxybutanoate: 9 g/L
Sodium dihydrogen phosphate: 4.0 g/L
Disodium hydrogen phosphate: 4.6 g/L
Potassium sulfate: 0.45 g/L
Magnesium sulfate: 0.39 g/L
Calcium chloride: 62 mg/L
Urea: 0.54 g/L
TE solution: 1.0 g/L
Composition of TE solution
Ferrous sulfate: 15 g/L
Manganese sulfate: 2.4 g/L
Zinc sulfate: 2.4 g/L
Copper sulfate: 0.48 g/L

### [Resin Composition Comprising Polyhydroxyalkanoic Acid (PHA)]

The polyhydroxyalkanoic acid (PHA) of the present invention can also be mixed with another additive to form a resin composition as long as the physical properties are not impaired. Examples of the other additive to be used include a resin other than the polyhydroxyalkanoic acid (PHA) of the present invention, an antioxidant, an ultraviolet absorber, a plasticizer, a flame retardant, an inorganic filler, and a crystal nucleating agent.

Examples of the resin other than the polyhydroxyalkanoic acid (PHA) of the present invention include a thermoplastic resin, a thermosetting resin, a polyolefin resin such as polyethylene and polypropylene, polyimide, polyamide, polyphenylene ether, polyether ketone, polyether ketone ketone, polybutadiene, polystyrene, polyester, polylactic acid, a phenol resin, poly(meth)acrylic acid, and a norbornene resin. Among them, a biodegradable resin is desirable.

### [Microparticle Comprising Polyhydroxyalkanoic Acid (PHA)]

The microparticles comprising polyhydroxyalkanoic acid (PHA)according to the present invention can take various shapes, such as sphere, plate, spindle, and needle, among which the spherical shape is preferred.

In addition, the microparticles comprising the polyhydroxyalkanoic acid (PHA) according to the present invention each have a particle size of 0.2 um to less than 10 um, preferably 7 um or less, considering the use in medical applications. Note that the particle size of microparticles comprising the polyhydroxyalkanoic acid (PHA) according to the present invention was measured using SEM observation images, processing by software (ImageJ), and dynamic light scattering, all of which will be described in detail below.

Furthermore, the microparticles comprising the polyhydroxyalkanoic acid (PHA) according to the present invention may have a porous form to increase the surface area.

The microparticles comprising the polyhydroxyalkanoic acid (PHA) according to the present invention are able to retain other substances not only on the surfaces but also inside the microparticles. Examples of the other substances include, but are not particularly limited to, an inorganic powder substance such as calcium carbonate, aluminum oxide, magnesium oxide, magnesium carbonate, mica, talc, and silica; an organic powder substance such as magnesium stearate and zinc stearate; and a solvent-soluble substance, as long as the characteristics of the microparticles comprising the polyhydroxyalkanoic acid according to the present invention are not impaired.

The microparticles comprising the polyhydroxyalkanoic acid (PHA) according to the present invention may have a 10% compression strength of 0.23 to 2.20 MPa in an aspect. The 10% compressive strength of the microparticles according to the present invention can be adjusted for example, by mixing in a resin such as cellulose.

In addition, the microparticles comprising the polyhydroxyalkanoic acid (PHA) according to the present invention include an aspect that can be dispersed in an aqueous solvent. Although water is given as an example of the aqueous solvent, the aqueous solvent is not limited thereto. Examples thereof include a mixed solvent of water and a hydrophilic solvent such as alcohol. The microparticles comprising the polyhydroxyalkanoic acid (PHA) according to the present invention is dispersible in an aqueous solvent and thus expected to have an effect that can be applied to various applications.

### [Production of Microparticle Comprising Polyhydroxyalkanoic Acid (PHA)]

As a method for producing microparticles from the polyhydroxyalkanoic acid (PHA) according to the present invention, various methods such as a spray drying method and a dispersion method can be applied, but an electrospray deposition method is preferred because this method can be applied to many types of the polyhydroxyalkanoic acid (PHA), the particle size and strength of the microparticles can be varied in a wide range, and the production process is simple. The electrospray deposition method (ESD method) will be described below, but the method for producing microparticles from the polyhydroxyalkanoic acid (PHA) according to the present invention is not limited thereto.

### [Electrospray Deposition Method]

The principle of an electrospray deposition method (ESD method) used in the specific embodiment of the present invention and an electrospray deposition device (ESD: electrospray device) used to carry out the electrospray deposition method will be described.

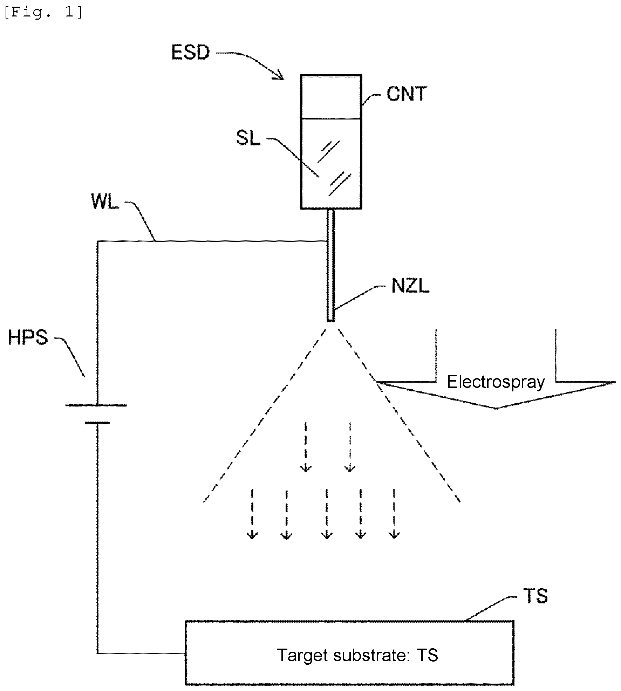

Figure 1 is a conceptual diagram showing a basic configuration of an electrospray deposition device. As shown in the figure, a container CNT contains a sample solution SL. The sample solution SL is, for example, an organic polymer solution or a polymer solution. In the present embodiment, the sample solution is a polyhydroxyalkanoic acid (PHA) solution dissolved in a solvent or a silica microparticle dispersion.

Although the ESD method is based on a very complicated physical phenomenon and all of the processes are not explained, the ESD method is generally considered the following phenomenon. The sample solution is contained in a thin capillary shaped nozzle NZL, and voltage of thousands to tens of thousands of volts is applied to a target substrate TS (counter electrode) opposing thereto. At a capillary tip, a strong electric field occurs due to an effect of electric field concentration, and microdroplets with charge on a liquid surface gather to form a cone (also called Taylor cone). The sample solution from the tip destroys surface tension to become a jet. The jet is strongly charged and becomes spray by a repulsion of electrostatic force (coulomb explosion). The droplets formed by spray are so small that the solvent is evaporated and dried within a short time to become fine nanoparticles or nanofiber. Of course, the solvent may be deposited in a wet state which is not evaporated or dried. The charged fine nanoparticles or nanofiber having a small diameter is pulled to the target substrate TS functioning as a counter electrode by electrostatic force. A pattern to be deposited may be controlled by an insulator mask or an auxiliary electrode, each of which is not shown. The sample is not limited to a solution as long as it is in a liquid state, and a dispersion solution is fine.

Preferably, the sample solution in the container CNT applies extrusion pressure toward the nozzle NZL by an air pressure syringe pump, a plunger, or the like (ejection means, not shown). The extrusion pressure is imparted, for example, by a stepping motor and a screw feed mechanism (not shown). The sample solution SL to which the extrusion pressure is applied has increased internal pressure in the container CNT so as to be discharged from the tip of the nozzle NZL. As described above, by installing an adjustment mechanism (the stepping motor and the screw feed mechanism) to adjust the speed of ejecting the sample solution, it is possible to appropriately adjust the ejection speed.

The nozzle NZL is made of metal, and positive voltage is supplied from a high voltage power supply HPS through a conductive wire WL. The negative side of the high voltage power supply HPS is connected to a target substrate TS (substrate serving as a counter electrode). By applying voltage from the high voltage power supply HPS, positive voltage is applied via the nozzle NZL to the sample solution SL so that the solution is positively charged. Note that the polarity of the voltage applied to the sample solution SL may be negative.

To implement the present invention, it is necessary to prepare only microparticles by suppressing the formation of nanofiber, control the particle size and physical properties, and adjust the selection of a sample and a solvent, concentration of a sample solution, high and low voltage, spray distance, environmental conditions such as temperature and humidity, for example.

The sprayed material becomes fiber or droplets and repeats division while scattering by repulsion due to charging to form nanofiber or nanoparticles. Since the sprayed material has a large surface area in a nano size, it is in an almost dried state when the sprayed material comes into contact with the substrate or a receiving tank. The shape or size may be changed depending on the spray conditions, and for example, when a polymer solution is used, thick nanofiber is formed with a high molecular weight and a high concentration, and thin nanofiber or nanoparticles are formed with a low molecular weight and a low concentration. Besides, various conditions such as voltage or a distance between the nozzle and the substrate and ambient temperature or humidity have an influence thereon. In the present embodiment, various solvent-soluble polyhydroxyalkanoic acids are used as samples to prepare microparticles under various conditions, and confirmation of the particle size, shape, and surface shape of the microparticles were each carried out by the method described in Examples. Not only the above-described device but also another type of ESD device can be used as the electrospray deposition device, and a method using air current described in Domestic Re-Publication of PCT International Publication No. WO 2009/060898 is preferred, especially for the purpose of mass production.

In order to produce the microparticles comprising the polyhydroxyalkanoic acid (PHA) according to the present invention, it is important to select an appropriate solvent. The solvent is not particularly limited as long as it sufficiently dissolves the PHA polymer, more strongly suppresses the formation of nanofibers, accelerates the formation of microparticles, and has a useful effect of changing the particle size. In the following Examples, chloroform and dimethyl carbonate were used as suitable solvents from this point of view.

### [Method of Recovering Microparticle Comprising Polyhydroxyalkanoic Acid (PHA)]

In a method of recovering the produced microparticles, it is possible to form a nanofiber structure at a landing site in the case where a product produced from the polyhydroxyalkanoic acid (PHA) is a nanofiber, but in the case where a product produced is a microparticle, it is necessary to recover the microparticle from the landing site without adhesion. Therefore, depending on the environmental conditions at the landing site, it is necessary to recover polyhydroxyalkanoic acid (PHA) not on a solid surface but in a liquid, depending on the physical properties of the polyhydroxyalkanoic acid (PHA) and the solvent used.

As a specific method of recovering microparticles, it is necessary to recover microparticles without binding to each other due to surface potential or the like, and when the produced microparticles were recovered using tap water, it was found that particles were bound to each other due to the influence of surface potential or the like. Thus, recovery using ethanol is preferred to recover microparticles in a dispersed state without binding to each other. Although absolute ethanol can be used as ethanol, depending on the microparticles to be produced, it is also possible to use the ethanol after dilution with water at around 10 to 30%. Note that even with tap water, it is also possible to recover microparticles in a dispersed state without binding to each other by appropriately blending an anionic surfactant or the like.

In order to recover the dried particles, vacuum drying is preferred when ethanol is used for recovery, but the recovery may also be achieved by natural drying. Vacuum drying is preferred when tap water containing a surfactant is used for recovery.

### [Method of Measuring Particle Size of Microparticle Comprising Polyhydroxyalkanoic Acid (PHA)]

### (1) SEM Observation Image and Processing by Software (ImageJ)

A measurement method of determining particle size by processing images obtained by SEM with ImageJ, which is an open-source and public-domain image processing software that is widely used for image analysis in scientific research and has become the de facto standard analysis tool in biological sciences. Processing with ImageJ makes it possible to calculate particle size from SEM photographs. The specific procedures are the following: 1. image input, 2. automatic binarization, 3. elimination of unnecessary particles, 4. binary image hole filling, and 5. measured results. As a result of the examination, this method is suitable to be used especially in the case of microparticles of several micrometers or more, and the accuracy of the measured results by this method was confirmed by measuring particle size through both this method and dynamic light scattering using microparticles of several hundred nm order obtained from P (3-HB-co-4-HB). As a result, as described in Example 4 below, the measured results obtained by both methods were almost the same. In particular, considering that the particle size is very small, less than 500 nm, the accuracy of the measured results by this method is judged to be very high.

### (2) Dynamic Light Scattering

There are many other methods for particle size (grain size) measurement of microparticles other than those mentioned above. Electrical detector method, centrifugal sedimentation method, laser diffraction method, FFF method, and the like are used, and each of them has its own characteristics. However, only dynamic light scattering is said to be able to measure the size at the electron microscopic measurement level and is utilized for ultrafine particle measurement. In this development, dynamic light scattering was used for grain size measurement of microparticles at the submicron level.

The device used was HORIBA NANO PARTICLE ANALYZER SZ-100, and the measurement conditions were as follows:
Detection angle: 90
Holder temperature: 25.0°C
Sample refractive index: 1.500-0.000i
Dispersion medium file: water
Dispersion medium refractive index: 1.333
Viscosity of dispersion medium: 0.896 mPa·s
Molecular form (dispersity): polydisperse
Particle size standard: scattered light intensity
Count rate: 1,285 kCPS

The present invention will be described in more detail below with Examples. Note that the present invention is in no way limited thereto.

### Examples

### [Example 1] Production of Microparticle from P (3-HB)

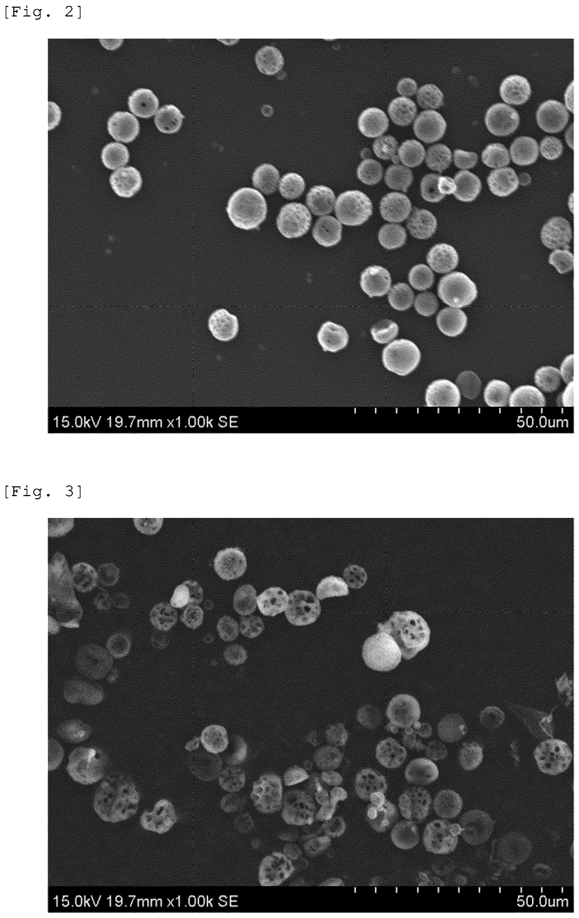

First, 1.5 g of a P (3-HB) resin-consisting of 3-HB without 3-HH as a repeating unit of polyhydroxyalkanoic acid (PHA) was dissolved in chloroform to prepare 100 g of a test sample solution with a concentration of 1.5% by wight. In a container CNT of a glass syringe (1 ml white hard syringe from Tsubasa Industry Co., Ltd.) attached with a metal double nozzle NZL (DN-24G from Musashi Engineering Inc.) with an internal diameter of 0.29 mm as shown in Figure 1, 1 mL of the test sample solution was placed, and the container was mounted on an electrospray deposition device (EsprayerES 2000, manufactured by Fuence Co., Ltd.). A 8.9 g container made of iron with a thickness of 0.25 mm, a diameter of 5 cm, and a height of 1 cm was placed on a target substrate TS (collector substrate), and 15 ml of absolute methanol was injected into the inside. The conditions of the electrospray at that time were a voltage between the nozzle NZL and the collector (target substrate TS) of 25 KV, a distance between the nozzle and the collector of 4 cm, and a liquid feeding flow rate of 20 µl/min. The substrate was scanned uniformly in the front-rear and left-right directions to spray and disperse the whole to obtain microparticles of P (3-HB). Note that a liquid containing microparticles of P (3-HB) was obtained under the same conditions except that the solution concentration was adjusted to 0.7 to 3.0% by weight. The microparticles shown in Figure 2 were obtained by drying the liquid. The average particle size was determined from the SEM observation images through particle size analysis of ImageJ, and the average particle size was 6.70 µm.

### [Example 2] Production of Porous Microparticle from P (3-HB)

First, 1.5 g of a P (3-HB) resin consisting of 3-HB without 3-HH as a repeating unit of polyhydroxyalkanoic acid (PHA) was dissolved in chloroform to prepare 100 g of a test sample solution with a concentration of 1.5% by wight. In a container CNT of a glass syringe (1 ml white hard syringe from Tsubasa Industry Co., Ltd.) attached with a metal nozzle NZL (27G from Musashi Engineering Inc.) with an internal diameter of 0.21 mm as shown in Figure 1, 1 mL of the test sample solution was placed, and the container was mounted on an electrospray deposition device (EsprayerES 2000, manufactured by Fuence Co., Ltd.). A 11.5 g container made of aluminum with a thickness of 0.5 mm, a length of 8 cm, a width of 10 cm, and a height of 5 mm was placed on the target substrate TS (collector substrate), and 15 ml of anhydrous ethanol was injected into the inside to be used for the recovery of microparticles. The conditions of the electrospray at that time were a voltage between the nozzle NZL and the collector (target substrate TS) of 25 KV, a distance between the nozzle and the collector of 2.5 cm, and a liquid feeding flow rate of 10 µl/min. The substrate was scanned uniformly in the front-rear and left-right directions to spray the entire surface, thereby obtaining dispersed 3-PHB (0% PHA) microparticles. Note that a liquid containing microparticles consisting of P (3-HB) was obtained at a solution concentration of 0.7 to 3.0% by weight under the conditions of other nozzle diameters and flow rates. The microparticles shown in Figure 3 were obtained by drying the liquid. The SEM observation image confirmed that the microparticles were spherical with porous surfaces. In addition, the particle size was measured using the SEM observation image and software (ImageJ) in the same manner as in Example 1, and the average particle size was about 6.4 um. The physical properties of the resin itself are considered to be related to the porosity, and the generation of porous microparticles was not observed when the content ratio of 3-hydroxyhexanoic acid (3-HH) as the repeating unit constituting the polyhydroxyalkanoic acid (PHA) according to the present invention was large. For example, the generation of porous microparticles was hardly observed in P (3-HB-co-3-HH) comprising 27% of the 3-hydroxyhexanoic acid (3-HH).

### [Example 3] Production of Microparticle from P (3-HB-co-3-HH)

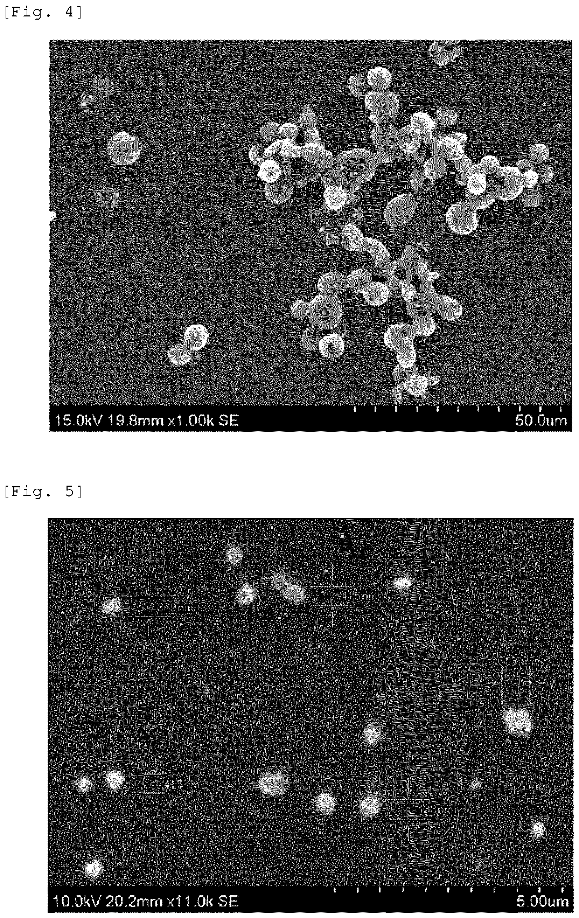

First, 1.5 g of a P (3-HB-co-3-HH) resin comprising 27% of 3-hydroxyhexanoic acid (3-HH) as a repeating unit was dissolved in chloroform to prepare 100 g of a test sample solution with a concentration of 1.5% by weight. In a container CNT of a glass syringe (1 ml white hard syringe from Tsubasa Industry Co., Ltd.) attached with a metal nozzle NZL (SNA-22G from Musashi Engineering Inc.) with an internal diameter of 0.42 mm as shown in Figure 1, 1 mL of the test sample solution was placed, and the container was mounted on an electrospray deposition device (EsprayerES 2000, manufactured by Fuence Co., Ltd.). A 8.9 g container made of iron with a thickness of 0.25 mm, a diameter of 5.5 cm, and a height of 1 cm was placed on a target substrate TS (collector substrate), and 15 ml of 90% ethanol was injected into the inside. The conditions of the electrospray at that time were a voltage between the nozzle NZL and the collector (target substrate TS) of 25 KV, a distance between the nozzle and the collector of 5 cm, and a liquid feeding flow rate of 20 µl/min. The substrate was scanned uniformly in the front-rear and left-right directions to spray and disperse the whole to obtain microparticles of P (3-HB-co-3-HH). Note that a liquid containing microparticles of P (3-HB-co-3-HH) was also obtained under the same conditions (metal nozzles 24G, 21G, and DN-24) except that the solution concentration was adjusted to 0.7 to 3.0% by weight. The microparticles shown in Figure 4 were obtained by drying the liquid. The SEM observation image showed that the shape of the microparticles was almost spherical, and porosity was hardly observed. The particle size was measured using the SEM observation image and software (ImageJ) in the same manner as in Example 1, and the average particle size was about 6.6 µm.

### [Example 4] Production of Microparticle from P (3-HB-co-4-HB)

First, 1.5 g of a P (3-HB-co-4-HB) resin containing 42% of 4-hydroxybutanoic acid (4-HB) as a repeating unit was dissolved in dimethyl carbonate to prepare 150 g of a test sample solution with a concentration of 1.0% by weight. In a container CNT of a glass syringe (1 ml white hard syringe from Tsubasa Industry Co., Ltd.) attached with a metal nozzle NZL (DN-24G from Musashi Engineering Inc.) with an internal diameter of 0.29 mm as shown in Figure 1, 1 mL of the test sample solution was placed, and the container was mounted on an electrospray deposition device (EsprayerES 2000, manufactured by Fuence Co., Ltd.). A 8.9 g container made of iron with a thickness of 0.25 mm, a diameter of 5 cm, and a height of 1 cm was placed on a target substrate TS (collector substrate), and 15 ml of tap water (five drops of surfactants in 50 ml) was injected into the inside. The conditions of the electrospray at that time were a voltage between the nozzle NZL and the collector (target substrate TS) of 25 KV, a distance between the nozzle and the collector of 5 cm, and a liquid feeding flow rate of 20 µl/min. The substrate was scanned uniformly in the front-rear and left-right directions to spray and disperse the whole to obtain microparticles of P (3-HB-co-4-HB). Note that a liquid containing microparticles of P (3-HB-co-4-HB) was also obtained under the same conditions except that the solution concentration was adjusted to 0.7 to 3.0% by weight. The microparticles shown in Figure 5 were obtained by drying the liquid.

The particle size of the obtained microparticles was measured using a SEM observation image, software (ImageJ) processing, and a dynamic scattered light intensity method under the measurement conditions described above.

As a result, the particle size measured by the SEM observation image and software (ImageJ) processing was 0.42 um as an average particle size. Meanwhile, the mode size measured by dynamic scattering light intensity method was 0.34 um (335.1 nm). Therefore, the particle size of 420 nm (0.42 µm) measured by the SEM observation image and software (ImageJ) processing and the mode size of 335.1 nm measured by the dynamic scattering light intensity method showed very close results, which confirmed that the measurement of particle size using an SEM observation image and software (ImageJ) processing was a reliable measurement method.

### [Example 5] Production of Microparticle Retaining Other Compound

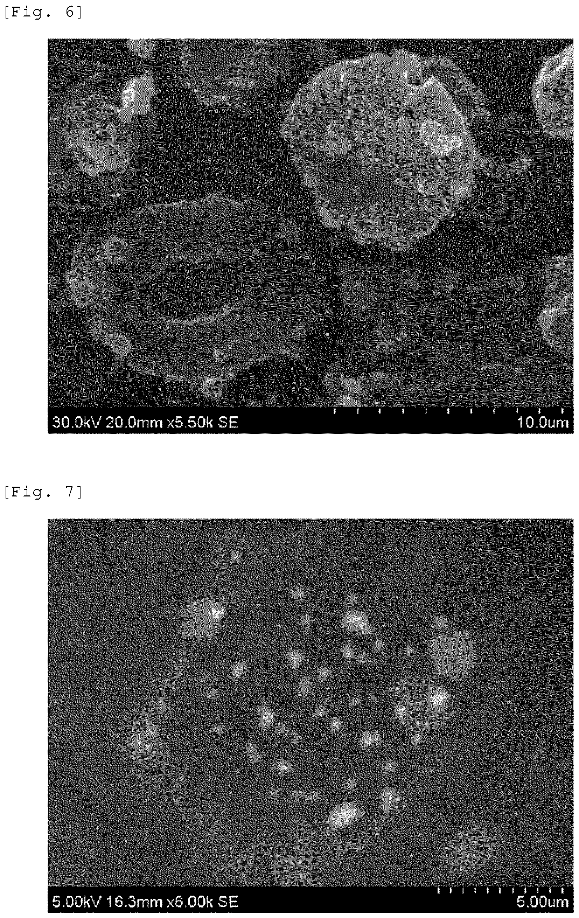

First, 1.5 g of a P (3-HB) resin consisting of 3-HB as a repeating unit of polyhydroxyalkanoic acid (PHA), to which silica particles, ADMAFINE SC2500-SPJ (manufactured by ADMATECHS COMPANY LIMITED), were added in an amount of 0.5% relative to its weight, was dissolved with chloroform to prepare about 100 g of a solution of 1.5% by weight with P (3-HB) . In a container CNT of a glass syringe (1 ml white hard syringe from Tsubasa Industry Co., Ltd.) attached with a metal nozzle NZL (DN-24G from Musashi Engineering Inc.) with an internal diameter of 0.29 mm as shown in Figure 1, 1 mL of the test sample solution was placed, and the container was mounted on an electrospray deposition device (EsprayerES 2000, manufactured by Fuence Co., Ltd.). A 8.9 g container made of iron with a thickness of 0.25 mm, a diameter of 5 cm, and a height of 1 cm was placed on a target substrate TS (collector substrate), and 15 ml of absolute alcohol was injected into the inside. The conditions of the electrospray at that time were a voltage between the nozzle NZL and the collector (target substrate TS) of 25 KV, a distance between the nozzle and the collector of 4 cm, and a liquid feeding flow rate of 20 µl/min (10 µl/min per nozzle because of a double nozzle). The substrate was scanned uniformly in the front-rear and left-right directions to spray the entire surface, thereby obtaining a dispersion of microparticles of P (3-HB) dispersed therein. The microparticles were naturally dried to obtain the microparticles shown in Figure 6. The microparticles each had a particle size of about 6 to 10 um and retained the silica particles on the surface thereof.

The P (3-HB) microparticles with retained silica particles described above were observed by a method described below to confirm that such silica particles were also retained inside the P (3-HB) microparticles.

Ultra-thin sectioning was performed according to the following procedure to make SEM observation.
1) Place the sample on a glass slide.
2) Add a drop of embedding resin on top of the powder to cure the resin by using EPON812 (epoxy resin) for 48 hours at 60°C.
3) Overlay the cured resin with a beam capsule filled with the embedding resin.
4) Cure the embedding resin (60°C, 48 hours).
5) Warm the glass slide and peel off the beam capsule in which the embedding resin has cured.
6) Remove the cured resin from the beam capsule.
7) Locate and mark the powder with an optical microscope.
8) Trim the powder periphery
9) Load the sample onto an ultramicrotome and prepare the surface with a diamond knife.
10) Cut the sample to a height that can be placed on a sample stand.
11) Adhere the sample to a copper plate with conductive double-sided tape.
12) Conduct carbon shadowing.

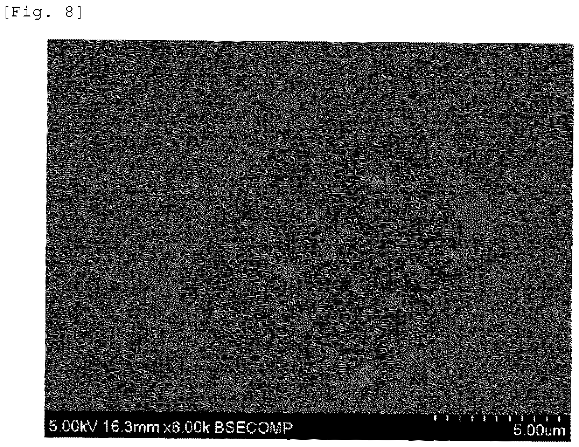

The SEM observation images are shown in Figure 7, and the observation image confirmed that particles corresponding to silica particles were also present within the P (3-HB) microparticles. Figure 8 shows an observation image of a BSE-SEM image. The BSE-SEM image is a reflection electron image of the SEM, a technique that makes it possible to observe the compositional distribution in the sample. This observation image confirmed that the P (3-HB) microparticles retained particles with chemical composition different from that of the P (3-HB) microparticles.

From the above results, it was confirmed that the silica particles were retained on the surface and inside the P (3-HB) particles after the electrospray deposition operation, although the strength of the microparticles to retain the silica particles was not clear, indicating that not only was polyhydroxyalkanoic acid (PHA) micronized by the electrospray deposition operation, but also other substances were retained and mixed into or integrated into the microparticles comprising the polyhydroxyalkanoic acid (PHA) according to the present invention by dissolving or dispersing those substances in the spray solution in the process. Hence, the microparticles comprising the polyhydroxyalkanoic acid (PHA) according to the present invention showed potential to be a useful carrier for other substances.

### [Example 6] Compressive Strength Measurement of Microparticle Containing Polyhydroxyalkanoic Acid (PHA)

Since fracture strength and deformation strength are practically important factors as physical properties possessed by microparticles when considering the use of such microparticles in various applications, a compression test was conducted on the microparticles comprising polyhydroxyalkanoic acid (PHA) according to the present invention to confirm the 10% compressive strength. The following were used as test samples:
Test sample (1): microparticles produced from P (3-HB) (microparticles produced in Example 1).
Test sample (2): microparticles produced from P (3-HB-co-3-HH) (microparticles produced in Example 3).

The compression test was performed using a Shimadzu micro compression tester MCT - 510 under conditions described below. Note that a very small amount of the test sample was scattered on a glass plate to conduct the compression test on each microparticle. The test results were evaluated on an average basis.

**[Table 1]**

| Test type | Compression test |
|---|---|
| Test force (mN) | 4.900 |
| Loading rate (mN/sec) | 0.0446 |
| Test particle size (µm) | 10±1 |
| Upper pressure indenter (µm) | Planar ϕ = 50 |
| Number of tests | 5 |

The following table shows results of the compression tests.

**[Table 2]**

| Sample No. | Sample name | Average particle size (µm) | Test force (mN) at 10% deformation | 10% Strength C(x) (MPa) |
|---|---|---|---|---|
| 1 | Test sample (1) | 10.47 | 0.31 | 2.20 |
| 2 | Test sample (2) | 10.27 | 0.03 | 0.23 |

From the above results, the test sample of microparticles comprising the polyhydroxyalkanoic acid (PHA) according to the present invention exhibited a 10% compressive strength of 0.23 to 2.20 MPa, and thus, it was confirmed that the microparticles had the strength to withstand the corresponding compressive strength. It was also confirmed that the microparticles produced from P (3-HB) showed higher values as 10% compressive strength than those produced from P (3-HB-co-3-HH) comprising 27% of 3-hydroxyhexanoic acid (3-HH) as a repeating unit, indicating higher strength. Therefore, the microparticles comprising the polyhydroxyalkanoic acid (PHA) according to the present invention can be used according to various use environments and applications by adjusting the blending ratio of 3-hydroxyhexanoic acid (3-HH) as its repeating unit.

### [Example 7] Thermal Property of Polyhydroxyalkanoic Acid (PHA) Polymer

Among the physical properties of polyhydroxyalkanoic acid (PHA), which is a raw material of the microparticle according to the present invention, the melting point is one of the important physical properties in view of the ease of processing the microparticle for use in practical applications. If it is possible to change the melting point, it can be an advantageous physical property in processing and the like. The melting point of P (3-HB), which has been relatively well studied, is reported to be 170 to 180°C in most cases, whereas the melting point of P (3-HB-co-3-HH), which comprises 27% of 3-hydroxyhexanoic acid (3-HH) as a repeating unit, is unknown, and the melting point of P (3-HB-co-4-HB) varies considerably depending on the literature. Therefore, the following method was used to confirm their melting points.

The measurement was performed using a differential scanning calorimeter DSC8500, manufactured by Perkin-Elmer, Inc., under measurement conditions of about 6 mg of the test sample in a nitrogen gas atmosphere at 5.00°C/min at an elevated temperature from 5.00°C to 200.00°C.

As a result, it was confirmed that the melting points of P (3-HB-co-3-HH) and P (3-HB-co-4-HB) were 79.8°C and 56°C, respectively.

### Industrial Applicability

The microparticle comprising polyhydroxyalkanoic acid (PHA) according to the present invention have, for example, excellent processability, biodegradability in a natural environment, biocompatibility, and biodegradability, with a wide range of melting points, particle size, and moderate compressive strength, and can be used for many industrial and medical applications without causing any environmental problems such as microplastics.

### Explanation of Letters or Numerals

- CNT: Container
- HPS: High voltage power supply
- NZL: Nozzle
- SL: Sample solution
- TS: Target substrate
- ESD: Electrospray deposition device
- WL: Wire

## Claims

1. A microparticle comprising polyhydroxyalkanoic acid (PHA), the microparticle comprising 3-hydroxybutanoic acid (3-HB) as a repeating unit of polyhydroxyalkanoic acid (PHA), and having a particle size of 0.2 um to less than 10 µm.

2. The microparticle according to claim 1, wherein the microparticle has a particle size of 7 um or less.

3. The microparticle according to claim 1 or 2, wherein the polyhydroxyalkanoic acid (PHA) further comprises 3-hydroxyhexanoic acid (3-HH) as a repeating unit thereof.

4. The microparticle according to claim 3, wherein a proportion of the 3-hydroxyhexanoic acid (3-HH) is 27% by weight or less with respect to a total weight of the repeating unit of polyhydroxyalkanoic acid (PHA).

5. The microparticle according to claim 3 or 4, wherein the polyhydroxyalkanoic acid (PHA) comprises a copolymer of the 3-hydroxybutanoic acid (3-HB) and the 3-hydroxyhexanoic acid (3-HH).

6. The microparticle according to any one of claims 1 to 5, wherein the polyhydroxyalkanoic acid (PHA) further comprises a unit of 4-hydroxybutanoic acid (4-HB) as a repeating unit thereof.

7. The microparticle according to claim 6, wherein a proportion of the 4-hydroxybutanoic acid (4-HB) is 40 to 50% by weight with respect to [a] total weight of the repeating unit of polyhydroxyalkanoic acid (PHA).

8. The microparticle according to claim 6 or 7, wherein the polyhydroxyalkanoic acid (PHA) comprises a copolymer of the 3-hydroxybutanoic acid (3-HB) and the 4-hydroxybutanoic acid (4-HB).

9. The microparticle according to any one of claims 1 to 8, wherein the polyhydroxyalkanoic acid (PHA) has an average molecular weight (Mw) of 100,000 to 1,300,000.

10. The microparticle according to any one of claims 1 to 9, wherein the polyhydroxyalkanoic acid (PHA) has a melting point of 55°C or more to 170°C or less.

11. The microparticle according to any one of claims 1 to 10, wherein the microparticle comprises a resin other than the polyhydroxyalkanoic acid (PHA).

12. The microparticle according to claim 11, wherein the resin other than the polyhydroxyalkanoic acid (PHA) is a biodegradable resin.

13. The microparticle according to any one of claims 1 to 12, wherein the microparticle is spherical.

14. The microparticle according to any one of claims 1 to 13, wherein the microparticle is porous.

15. The microparticle according to any one of claims 1 to 14, wherein the microparticle retains other substances on a surface and/or inside thereof.

16. The microparticle according to any one of claims 1 to 15, wherein the microparticle has a 10% compressive strength of 0.23 to 2.20 MPa.

17. The microparticle according to any one of claims 1 to 16, wherein the microparticle is dispersible in an aqueous solvent.

18. A method for producing the microparticle according to any one of claims 1 to 17, comprising:
Step 1: a step of providing a microorganism that produces polyhydroxyalkanoic acid (PHA);
Step 2: a step of proliferating the microorganism of Step 1 in a medium;
Step 3: a step of causing an animal to ingest a proliferated microorganism;
Step 4: a step of recovering and purifying the polyhydroxyalkanoic acid (PHA) from excreta of the animal of Step 3; and
Step 5: a step of making microparticles from the polyhydroxyalkanoic acid (PHA) obtained in Step 4.

19. The method for producing the microparticle according to claim 18, wherein Step 5 is a step of micronizing a resin composition comprising the polyhydroxyalkanoic acid (PHA) obtained in Step 4.
